# EUROPEAN PATENT APPLICATION

(11) **EP 2 800 052 A1**
(43) Date of publication of application: **05.11.2014**
(21) Application number: 12862298.2
(22) Date of filing: 26.12.2012
(51) Int. Cl.: G06Q 50/22, H04W 88/00

(54) **METHOD AND APPARATUS FOR MANAGING PERSONAL HEALTH**

(30) Priority: 28.12.2011 KR 20110144090; 06.01.2012 KR 20120001873
(71) Applicant: Samsung Electronics Co., Ltd., Suwon-si, Gyeonggi-do 443-742 (KR)
(72) Inventor: KIM, Do-Young, Gyeonggi-do 443-742 (KR); LIM, Nae-Hyun, Gyeonggi-do 443-742 (KR); KONG, Dong-Keon, Gyeonggi-do 443-742 (KR); CHANG, Yong, Gyeonggi-do 443-742 (KR); LIM, Hyoung-Kyu, Gyeonggi-do 443-742 (KR)
(74) Representative: Nederlandsch Octrooibureau
(86) International application number: PCT/KR2012/011444
(87) International publication number: WO 2013/100547

(57) **Abstract**

Disclosed are a method and an apparatus for managing personal health. A gateway capable of communicating with a health device and a non-health device sets up a communication link with a communication device according to a first communication protocol, and determines whether the health device requires a higher level of security. When the communication device is determined as a health device, the gateway executes a health security algorithm which provides a higher level of security that a security algorithm applied to the first communication protocol, and communicates with the health device by using the health security algorithm.

## Description

### Technical Field

The present invention relates to management of personal health, and more particularly to a method and an apparatus for safely managing personal health record by improving a security of the personal health record.

### Background Art

A hospital-centered medical record management system has a problem in that personal health information is scattered in many medical organizations such that it is not integrated but non-systematically operated, and a Personal Health Record (PHR) has been suggested to solve the problem. The personal health record is directed to collectively managing medical treatment information of persons provided from medical organizations and health records recorded by the persons themselves. If the personal health records are used, persons can be provided with medical services and perform self-management anytime and anywhere, and can be provided with an on-demand medical service suitable for the persons, that is, a healthcare service.

The healthcare service includes collecting and storing health data collected by various health devices such as a blood pressure monitor, a body temperature meter, a glucometer, and a blood analyzer. The health data is transmitted to an external medical service provider or provided to a user through a display unit such as a television (TV), a mobile phone, or a smart phone. In order to support the health care service more efficiently, a health gateway for collecting and storing health data from health devices located in or around a house may be provided in the house. The health gateway performs communications with health devices using a near field communication technology such as WiFi, Bluetooth, or ZigBee.

For more efficient use of communication devices, the health gateway may be collocated with a general gateway/access point (AP) supporting communication with a general device such as a mobile phone, a smartphone, a notebook including a communication module, and a smart TV instead of a health device. In another case, an algorithm supporting a healthcare service may be additionally provided in the gateway supporting a wireless near field communication technology.

The health data requires a high security as compared with non-health data in that they contain not only simple individual information but private health information and life information such as a disease history of a user. However, because health devices use an existing general communication protocol according to the related art, a security of the same level as that of general devices is applied so that the requirements cannot be required.

Accordingly, a technology for providing a high security for health devices has been required in an environment in which health devices and non-health devices coexist using the same communication protocol.

### Detailed Description of the Invention

### Technical Problem

The present invention provides a security method and a security apparatus for a health management system.

The present invention provides a method and an apparatus for providing a high security for health devices in an environment in which health devices and non-health devices coexist using the same communication protocol.

The present invention provides a method and an apparatus for reducing power consumption due to driving of an additional security algorithm for health devices.

The present invention provides an apparatus and a method for safely managing a personal health record by increasing a security of the personal health record.

The present invention provides an apparatus and a method for managing a personal health record by which a resource can be efficiently used by determining whether a security of a health measuring unit is set according to situations.

### Technical Solution

In accordance with an embodiment of the present invention, there is provided a method of providing a security by a gateway in a health system, the method including: setting a communication link according to a first communication protocol with a communication device; determining whether the communication device is a health device requiring a security of a high level; if the communication device is a health device, performing a health security algorithm providing a security of a level higher than that of a security algorithm applied in the first communication protocol; and performing a communication with the health device by using the health security algorithm.

In accordance with another embodiment of the present invention, there is provided a gateway apparatus for providing a security for a health system, the gateway apparatus including: a transceiver for performing a communication with at least one communication device; and a controller for, if a communication link according to a first communication protocol with the communication device is set, determining whether the communication device is a health device requiring a security of a high level, if the communication device is a health device, performing a health security algorithm providing a security of a level higher than that of a security algorithm applied in the first communication protocol, and performing a communication with the health device by using the health security algorithm.

In accordance with another embodiment of the present invention, there is provided a method of managing a health record, the method including: receiving first control information on whether security of at least one measurement apparatus is executed from a health record management server or a user; transmitting second control information on whether security of the at least one measurement apparatus is executed to the at least one measurement apparatus based on the first control information; and receiving data measured by the at least one measurement apparatus and generated based on the second control information and providing the data to the health record management server or the user.

In accordance with another embodiment of the present invention, there is provided a method of managing a health record, the method including: determining whether a security of at least one measurement apparatus is executed and generating control information on whether a security of the at least one measurement apparatus is executed; transmitting the control information to the at least one measurement apparatus; and receiving data measured by the at least one measurement apparatus and generated based on the control information.

In accordance with another embodiment of the present invention, there is provided a method of managing a health record, the method including: determining whether a security is executed on health data based on control information provided by a health record management server or a user; measuring the health data; executing a security algorithm to set a security in the measured health data according to the determination; and transmitting the data in which the security is set to the health record management server or the user.

In accordance with another embodiment of the present invention, there is provided an apparatus for managing a health record, the apparatus including: a transmitter that transmits a signal; a receiver that receives a signal; and a controller that transmits second control information on whether security of the at least one measurement apparatus is executed to the at least one measurement apparatus based on the first control information on whether security of at least one measurement apparatus is executed from a health record management server or a user, and receives data measured by the at least one measurement apparatus and generated based on the second control information and transmits the data to the health record management server or the user.

In accordance with another embodiment of the present invention, there is provided an apparatus for managing a health record, the apparatus including: a transmitter for transmitting a signal; a receiver that receives a signal; a controller for determining whether a security is executed on at least one measurement apparatus and generating control information on whether a security is executed, and transmitting the control information to the at least one measurement apparatus.

In accordance with another embodiment of the present invention, there is provided an apparatus for managing a health record, the apparatus including: a transmitter for transmitting a signal; a receiver that receives a signal; a controller for determining whether a security is executed on health data based on control information provided by a health record management server or a user, and executing a security algorithm to set a security in the measured health data according to whether a security is executed.

### Brief Description of the Drawings

FIG. 1 is a view exemplifying a configuration of a health system according to an embodiment of the present invention;
FIG. 2 is a message flowchart for explaining an execution procedure of a health security algorithm according to an embodiment of the present invention;
FIG. 3 is a message flowchart for explaining an ending procedure of a health security algorithm according to an embodiment of the present invention;
FIG. 4 is a flowchart showing an operation of executing a health security algorithm in a gateway according to an embodiment of the present invention;
FIG. 5 is a flowchart showing an operation of ending a health security algorithm in a gateway according to an embodiment of the present invention;
FIG. 6 is a block diagram showing a configuration of a gateway for executing a health security algorithm according to an embodiment of the present invention;
FIG. 7 is a view showing a personal health record management system according to an embodiment of the present invention;
FIG. 8 is a flowchart for explaining an operation of a personal health record management system according to an embodiment of the present invention;
FIG. 9 is a flowchart for explaining an operation of a personal health record management system according to another embodiment of the present invention;
FIG. 10 is a flowchart for explaining an operation of a personal health record management system according to another embodiment of the present invention;
FIG. 11 is a flowchart for explaining an operation of a personal health record management system according to another embodiment of the present invention;
FIG. 12 is a flowchart showing an operation of a health measurement control apparatus of a personal health record management system according to an embodiment of the present invention;
FIG. 13 is a flowchart showing an operation of a PHR server of a personal health record management system according to an embodiment of the present invention; and
FIG. 14 is a flowchart showing an operation of a measurement apparatus of a personal health record management system according to an embodiment of the present invention.

### Mode for Carrying Out the Invention

Hereinafter, a preferred embodiment of the present disclosure will be described with reference to the accompanying drawings. Further, in the following description of the present invention, a detailed description of known functions and configurations incorporated herein will be omitted when it may make the subject matter of the present invention rather unclear. The terms which will be described below are terms defined in consideration of the functions in the present disclosure, and may be different according to users, intentions of the users, or customs. Therefore, the definitions of the terms should be determined based on the contents throughout the specification.

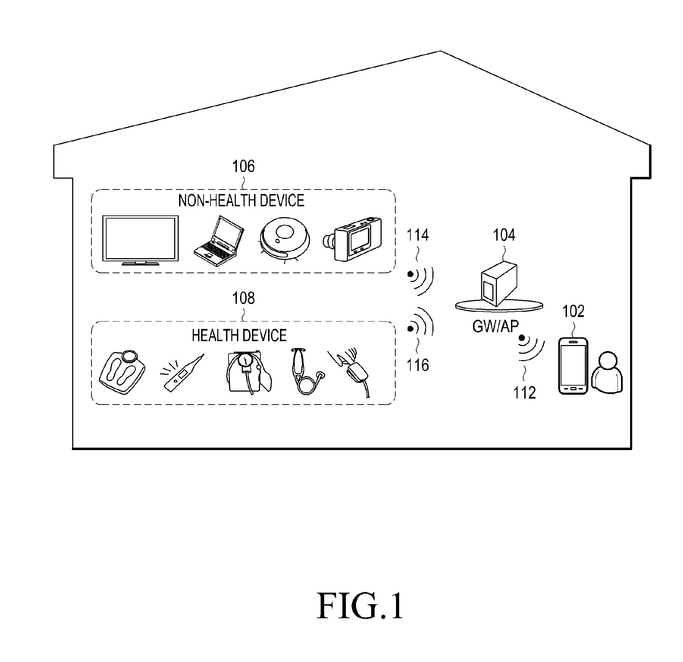

FIG. 1 exemplifies a configuration of a health system according to an embodiment of the present invention.

Referring to FIG. 1, there exists at least one health device 108 such as a scale, a glucometer, a blood pressure monitor, and a non-health device (also referred to as a general device) 106 such as a smart TV, a notebook including a communication module, and an home appliance including a communication module. The health device 108 and the non-health device 106 may perform a communication by using the same communication protocol, for example, WiFi, Bluetooth, or ZigBee. In the specification, an exemplary embodiment of the present invention will be described while taking communication through WiFi as an example.

A gateway/access point (hereinafter, a gateway) 104 may perform communication with the non-health device 106 and/or the health device 108 through WiFi, and remote control data or health data as information according to communication with the devices 106 and 108 are forwarded to a portable terminal 102 of a user and, if possible, a control command from the portable terminal 102 is forward to the devices 106 and 108. Here, the gateway 104 sets communication links 114, 116, and 112 with the non-health device 106, the health device 108, and the portable terminal 102 by using a WiFi communication protocol, and forwards the data and the command through the communication links. As a selectable embodiment, the gateway 104 may set communication links with the portable terminal 102 through the Internet and a mobile communication network to which the portable terminal 102 is subscribed.

The gateway 104, in particular, the gateway having an AP function may be opened to not only a registered user but a nonregistered user. In this case, a malicious nonregistered user may connect to the gateway 104 to steal private data by the health device 108. In particular, when the gateway 104 provides a security algorithm of the same level to all devices connected through WiFi, the problems may frequently occur. Thus, in the following embodiments of the present invention, when the health device 108 is connected, an operation for applying a security algorithm of a higher level, that is, a health security algorithm to the communication link 116 with the health device 108 is provided. Here, because the details of the health security algorithm deviate from the scope of the present invention that is sought to be protected, the health security algorithm will be omitted. Then, when a general security algorithm of WiFi is applied to the communication link 114 with the non-health device 106, a health security algorithm is not applied.

The health security algorithm may be added to a general security algorithm of WiFi to be performed. The general security algorithm specifically includes a security using an approach control based Service Set Identifier (SSID), and a security using a privacy based Wired Equivalent Privacy (WEP).

When the health devices and the non-health devices use the same type of communication protocol, that is, WiFi, the gateway 104 requires an additional operation to classify devices connected to the gateway 104, that is, to determine whether the connected devices are health devices.

As an embodiment, the gateway 104 stores information on a health device usable by the user in a database (DB) and manages the information. That is, before using a health device in a house for the first time, the user registers identification information on the health device, for example, a serial number of the device, a Media Access Control (MAC) address, and a WiFi version in the gateway 104. The DB may further include profile information such as the type of the health device, a manufacturer of the device, and the like together with the identification information on the health device.

As another embodiment, when transmitting a message requesting setting of a communication link to the gateway 104, the health device 108 transmits the profile information on the type of the device in the message. The profile information may simply indicate whether the device is a health device or may further include information on the type of the health device, the manufacturer of the device, and the like. The gateway 104 recognizes that the health device 108 is connected with reference to the profile information included in the message.

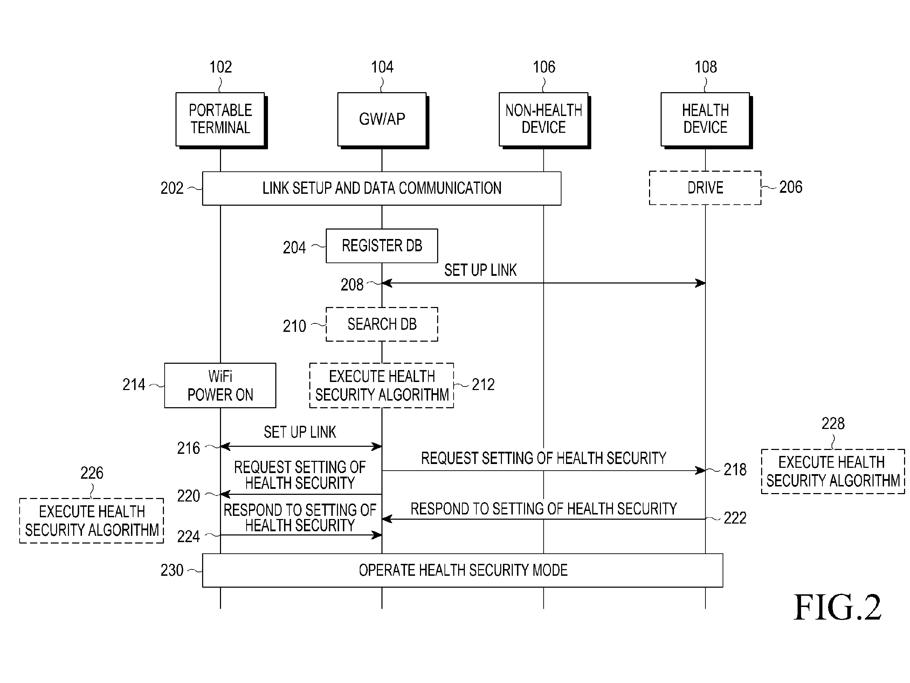

FIG. 2 is a message flowchart for explaining an execution procedure of a health security algorithm according to an embodiment of the present invention.

Referring to FIG. 2, in step 202, the gateway 104 sets a communication link with the non-health device 106 and the portable terminal 102 through WiFi, and performs data communication. In step 204, a preliminary registration of the health device 108, that is, an operation of storing identification information on the health device 108 in the DB of the gateway 104 is performed. Here, although it has been shown that step 204 of performing a preliminary registration of the health device 108 is performed after step 202, step 204 may be performed at any time before communication is started by the health device 108 irrespective of the performance of step 202. Further, step 202 may be omitted.

After being driven by switching on a power source in step 206, the health device 108 transmits a link setup message to the gateway 104 and receives a response message corresponding to the transmitted link setup message to set a communication link in step 208. As a selectable embodiment, during or after a step of setting the communication link, a message further including at least one of profile information on the health device 108 and information on an additional security algorithm may be transmitted from the health device 108 to the gateway 104. As a selectable embodiment, the health device 108 may specifically indicate a desired health security algorithm and security parameters.

When a DB storing preliminary registration information is present, the gateway 104 searches the DB for identification information on the health device 108 in step 210. The identification information may be acquired in step 208 of setting a link. If identification information on the health device 108 is present in the DB, the gateway 104 executes a predetermined security algorithm for providing a higher level of security for a health care service, that is, a health security algorithm in step 212. The health security algorithm is executed on a communication link between the gateway 104 and the health device 108, and may be determined according to a selection of the manufacturer or the designer in advance.

In step 218, the gateway 104 transmits a health security setting request message for requesting execution of a health security algorithm to the health device 108. In step 228, the health device 108 recognizes that a health security algorithm is executed by the health security setting request message in the gateway 104, and accordingly executes a health security algorithm. In step 222, the health device 108 transmits a health security setting response message that informs the gateway that the health security algorithm has been executed to the gateway 104. Thereafter, in step 230, communication of a health security mode protected by a health security algorithm is performed between the gateway 104 and the health device 108.

Meanwhile, when the user intends to connect to the health device 108 through the portable terminal 102 or identify the health data collected by the health device 108, a health security algorithm should be performed even in the portable terminal 102. Accordingly, the gateway 104 instructs execution of a health security algorithm to the portable terminal 102 through the following procedure.

The portable terminal 102 executes a WiFi communication module in step 214, and sets a communication link by WiFi with the gateway 104 in step 216. If recognizing that a communication link is set with the portable terminal 102 after a health security algorithm is executed in step 212, the gateway 104 transmits a health security setting request message to the portable terminal in step 220. After executing a health security algorithm in response to the health security request message in step 226, the portable terminal responds with the health security setting response message in step 224. Then, in step 230, communication protected by a health security algorithm is performed between the portable terminal 102, the gateway 104 and the health device 108. That is, in step 230, the health device 108 may communicate with the gateway 104, communicate with the portable terminal 102 through the gateway 104, or directly communicate with the portable terminal 102.

When another health device is connected, the same operation may be repeatedly performed on the other health device.

However, since a health security algorithm of a higher level is continuously executed only for health devices, power consumption may be excessively generated in the gateway 104. Thus, when the health device releases a communication link, it is necessary to complete execution of a health security algorithm.

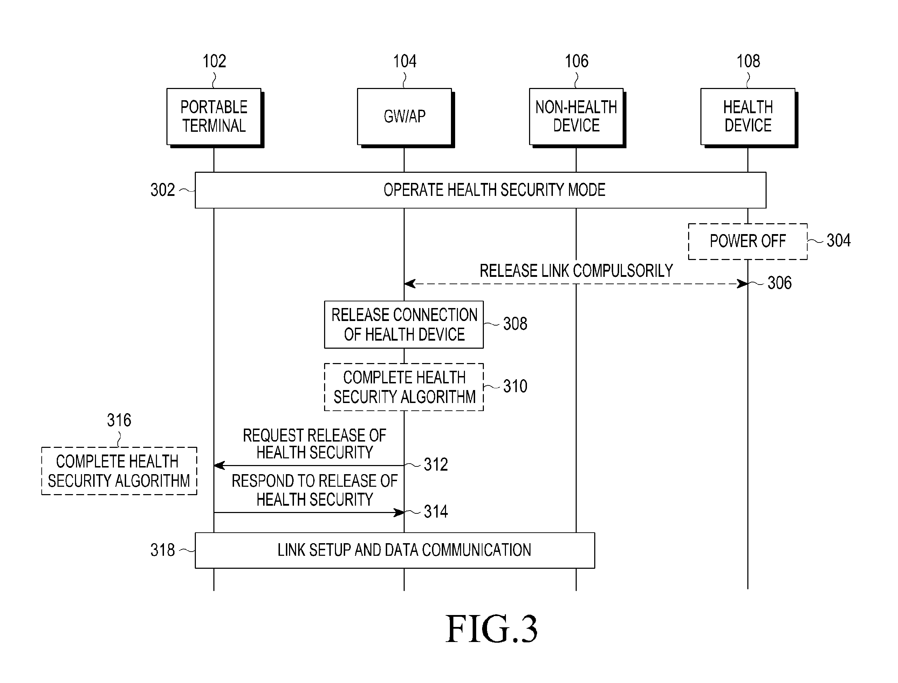

FIG. 3 is a message flowchart for explaining an ending procedure of a health security algorithm according to an embodiment of the present invention.

Referring to FIG. 3, the gateway 104, the health device 108, and the portable terminal 102 perform communication in a security mode protected by the health security algorithm. If a power source of the health device 108 is switched off in step 304, a communication link between the health device 108 and the gateway 104 is compulsorily released in step 306. As another embodiment, the health device 108 may request a release of the communication link from the gateway 104 before the power source of the health device 108 is switched off.

The gateway 104 detects that the communication with the health device 108 is released in step 308, and the health security algorithm which is being executed on the health device 108 is completed in step 310. As another embodiment, the gateway completes a health security algorithm when all the connected health devices release the communication link.

If the portable terminal 102 executes a health security algorithm, the gateway 104 transmits a health security release request message for instructing a completion of the health security algorithm to the portable terminal 102 in step 312, and the portable terminal completes the health security algorithm which is being executed on the health device 108 in response to the health security release request message in step 316. If the portable terminal 102 transmits a health security release response message to the gateway 104 in step 318, it is informed that the health security algorithm is completed.

Thereafter, in step 318, communication to which a general algorithm of WiFi is applied is performed between the gateway 104, the non-health device 106, and the portable terminal 102.

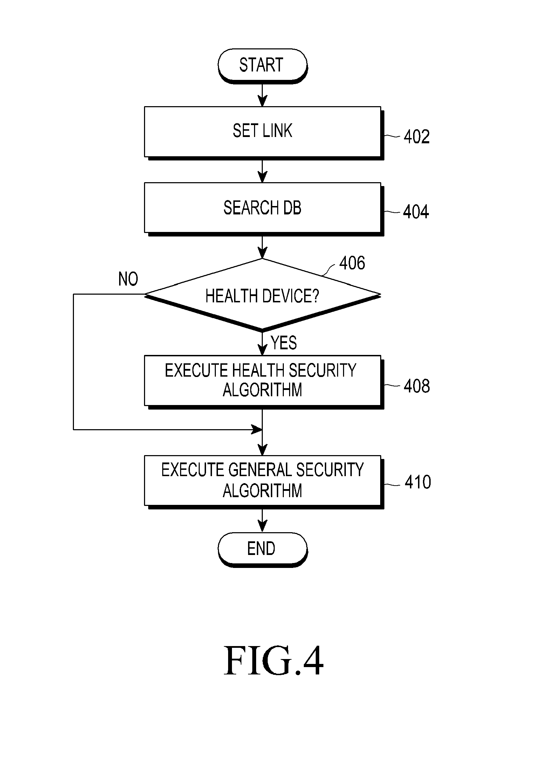

FIG. 4 is a flowchart showing an operation of executing a health security algorithm in a gateway according to an embodiment of the present invention. Although an embodiment in which a DB storing identification information of health devices is used has been described, it is apparent that a similar operation may be applied to a case in which a message including profile information is received from a health device. The shown operation may be performed irrespective of whether the non-health device is connected to the gateway.

Referring to FIG. 4, a communication link based on WiFi is set between the health devices and the communication device in step 402, and the gateway searches the DB for identification information of the registered health devices in step 404. In step 406, the gateway determines whether identification information on the communication device in which the communication link is set is stored in the DB. If the identification information is stored in the DB, the gateway determines that the communication device is a health device and executes a health security algorithm in step 408, and also requests the health device to execute a health security algorithm. Thereafter, the gateway proceeds to step 410. Meanwhile, if the identification information is not stored in the DB, the gateway determines that the communication device is a non-health device and executes a general security algorithm of WiFi in step 410. Thereafter, the health security algorithm or the health security algorithm and the general security algorithm provide a security to the communication between the gateway and the communication device. As a selectable embodiment, the health security algorithm may replace a general security algorithm of WiFi to be used.

Although it will not be shown, the gateway may instruct execution of a health security algorithm to the portable terminal according to selection of the user or a preliminary setting after the health security algorithm is executed in step 408. Accordingly, a health security algorithm is applied to communication between the portable terminal, and the gateway and the health device.

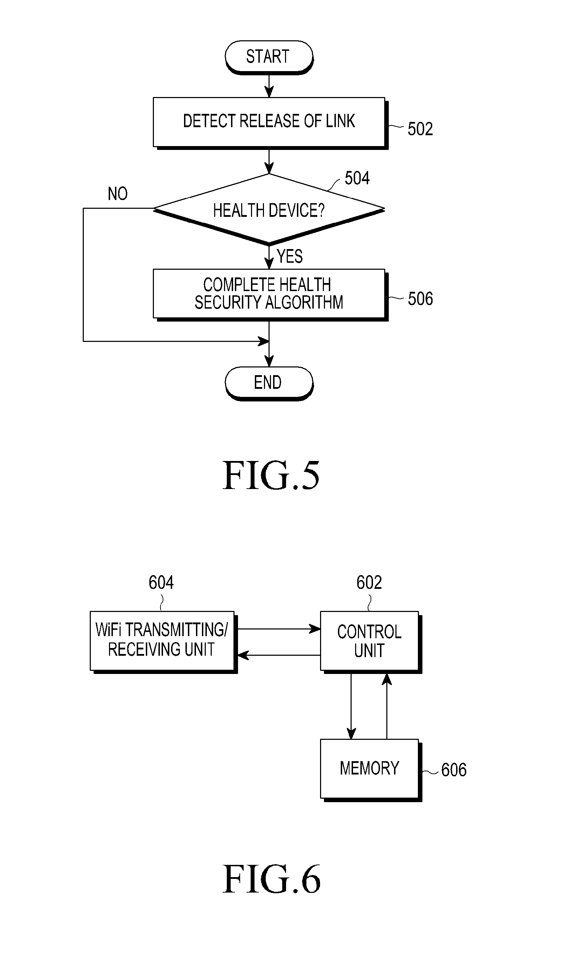

FIG. 5 is a flowchart showing an operation of ending a health security algorithm in a gateway according to an embodiment of the present invention.

Referring to FIG. 5, it is detected that a communication link set with the communication device based on WiFi is released in step 502, and it is determined that the communication device is a health device in step 504. The determination may be performed with reference to a search of the DB, profile information included in the message received from the health device, and a context stored with respect to a communication link. When it is determined that the communication device is a health device, the gateway completes a health security algorithm performed on the communication link or commonly applied to the communication with the health devices in step 506.

FIG. 6 is a block diagram showing a configuration of a gateway for executing a health security algorithm according to an embodiment of the present invention.

Referring to FIG. 6, the gateway performs communication according to a predetermined communication protocol such as WiFi, Bluetooth, or ZigBee with the portable terminal, the health devices, and the non-health devices through a transceiver 604. If it is detected that a communication link with a communication device is set by the transceiver 604, a controller 602 determines whether the communication device is a health device with reference to identification information of the health devices registered in a memory 606 in advance or profile information in the message received from the communication device through the transceiver 604. If it is identified that the communication device is a health device, the same health security algorithm is executed in the health device by executing a health security algorithm designated in advance and informing the health device of the execution of the health security algorithm through the transceiver 604. If the communication link with the health device is released, the controller 602 completes the health security algorithm to reduce power consumption.

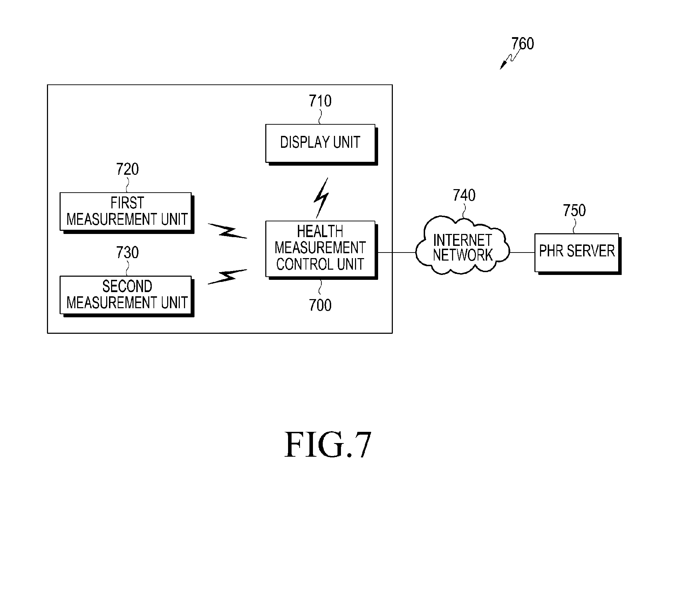

FIG. 7 is a view showing a Personal Health Record (PHR) management system according to another embodiment of the present invention.

Referring to FIG. 7, the personal health record management system 760 according to the embodiment of the present invention includes a display unit 710, first and second measurement units 720 and 730, a health measurement control unit 700, and a Personal Health Record (PHR) server 750. In an embodiment of the present invention, the display unit 710, the first and second measurement units 720 and 730, and the health measurement control unit 700 may be provided at home, and the PHR server 750 may be provided in a hospital.

The display unit 710 may be, for example, a portable terminal, a mobile phone, a Personal Digital Assistants (PDA), and a personal computer, and the first and second measurement units 720 and 730 are devices for measuring personal health, and may be health devices such as a scale, a glucometer, a blood pressure monitor, and a heart rate monitor. The health measurement control unit 700 is located at home, and may be a gateway for registering and managing the health devices. Although FIG. 7 shows only two measurement units 720 and 730, an additional measurement unit may be included. The first and second measurement units 720 and 730 include a security function, and a security algorithm may be executed according to setting of a security and the security algorithm may be different for the measurement units.

The health measurement control unit 700 controls execution of securities of the first and second measurement units 720 and 730 according to an instruction of the PHR server 750 or the display unit 710, and when the measurement units transmit measurement data while a security is executed on the measurement data, the measurement data may be provided to the display unit 710 after the security of the measurement data is released. Here, execution of security means execution of encryption and the like, and releasing of the security means that plain data are generated by releasing encryptions (that is, decryption).

The PHR server 750 stores a personal health record collected through the measurement units and may determine execution of the security of the measurement units, and accordingly, the measurement units may be registered in the PHR server 750 and the PHR server 750 may store information on the security algorithms of the measurement units.

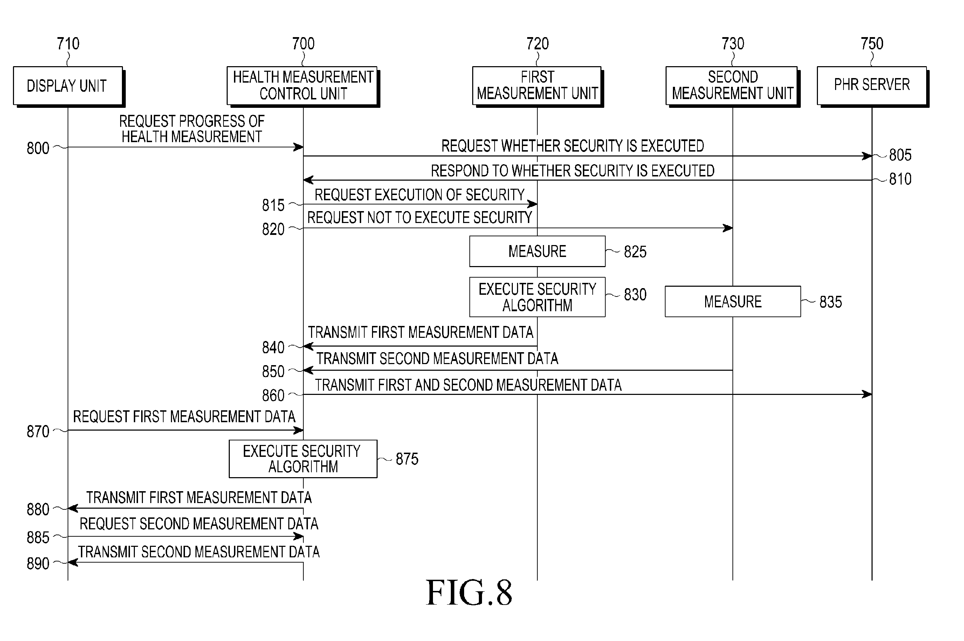

FIG. 8 is a flowchart for explaining an operation of a personal health record management system according to an embodiment of the present invention.

Referring to FIG. 8, the user transmits a health measurement progress request related to the first and second measurement units 720 and 730 to the health measurement control unit 700 through the display unit 710 (800). In response to the request, the health measurement control unit 700 requests information on execution of a security of the first and second measurement units 720 and 730 from the PHR server 750 (805). The PHR server 750 determines execution of securities of the measurement units according to the situation and transmits the determined information to the health measurement control unit 700 while the information is carried in the response to the execution of a security (810), and for example, when health data measured by the first measurement unit 720 are important data as compared with the second measurement unit 730, the PHR server 750 may determine that the first measurement unit 720 executes a security and the second measurement unit 730 does not execute a security. Then, the PHR server 750 may determine execution of securities of the measurement units in consideration of the health state of the user, the type of the measurement units, purposes of health measurements, and the like.

The PHR server 750 may transmit security related information on the measurement unit which has been determined to execute a security when execution of a security is responded to the health measurement control unit 700. The security related information may include, for example, the type of a security algorithm which will be applied to the security of data and/or an input parameter thereof. This is because a security of measured data can be released such that the user recognizes the data from the display unit 710 when data measured by the health measurement control unit 700 is provided to the display unit 710, and when security related information on the measurement units is stored in the health measurement control unit 700 in advance, transmission of separate security related information may be omitted.

If receiving a response on execution of a security, the health measurement control unit 700 transmits a message controlling execution of securities of the measurement units to the measurement units (815 and 820). For example, the first measurement unit 720 is requested to execute a security (815), and the second measurement unit 730 is requested not to execute a security (820). If data on the health of the user is measured (825), the first measurement unit 720 executes a security algorithm on the data (830), and transmits the first measurement data to the health measurement control unit 700 (840). Because the second measurement unit 730 does not execute a security, it measures data on the health of the user (835), and transmits second measurement data to the health measurement control unit 700 without executing a security algorithm on the measured data (850).

If receiving the first and second measurement data, the health measurement control unit 700 transmits the data to the PHR server 750, and transmits the corresponding data to the user if the user makes a request (860). Then, if the user requests first measurement data (870), a security algorithm is executed on the first measurement data to release a security (875) because a security is executed on the first measurement data such that the data whose security is released is transmitted to the display unit 710 (880). When the user requests the second measurement data (885), the second measurement data is transmitted to the display unit 710 while a security algorithm is not executed because a security is not executed on the second measurement data.

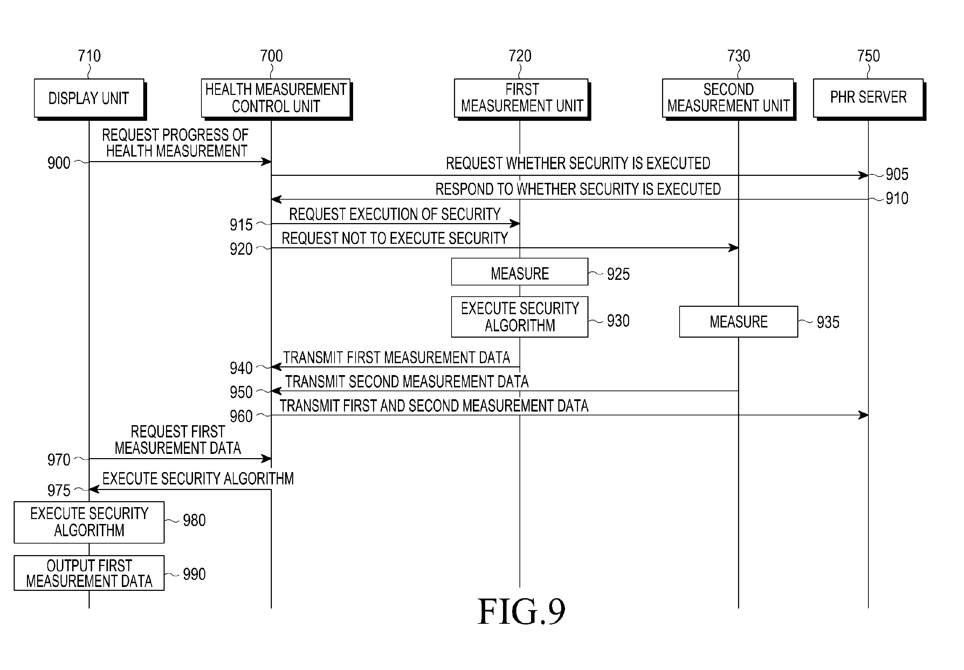

FIG. 9 is a flowchart for explaining an operation of a personal health record management system according to another embodiment of the present invention.

Referring to FIG. 9, if the user transmits a health measurement progress request related to the first and second measurement units 720 and 730 to the health measurement control unit 700 through the display unit 710 (900), the health measurement control unit 700 requests information on execution of a security of the first and second measurement units 720 and 730 from the PHR server 750 (905). The PHR server 750 transmits a security execution response including information on execution of a security in which a security is executed on the first measurement unit 720 and a security is not executed on the second measurement unit 730 to the health measurement control unit 700 (910). Then, the PHR server 750 may transmit security related information on the first measurement unit 720 to the health measurement control unit 700. The security related information may include, for example, information on a security algorithm executed by the first measurement unit 720.

If receiving a security execution response, the health measurement control unit 700 requests the first measurement unit 720 to execute a security (915) and the second measurement unit 730 not to execute a security (920). If data on the health of the user is measured (925), the first measurement unit 720 executes a security algorithm on the data (930), and transmits the first measurement data to the health measurement control unit 700 (940). Because the second measurement unit 730 does not execute a security, it measures data on the health of the user (935), and transmits second measurement data to the health measurement control unit 700 without executing a security algorithm on the measured data (950).

If receiving the first and second measurement data, the health measurement control unit 700 transmits the data to the PHR server 750 (960), and transmits the corresponding data to the user if the user makes a request. Then, if the user requests first measurement data (970), security algorithm related information as well as the first measurement data may be transmitted to the display unit 710 to release a security because a security is executed on the first measurement data (975). Then, when a security algorithm for the measurement units is stored in the display unit 710, transmission of the security algorithm related information may be omitted. If receiving the first measurement data, the display unit 710 releases a security by executing a security algorithm on the first measurement data (980) and outputs the data whose security is released (990).

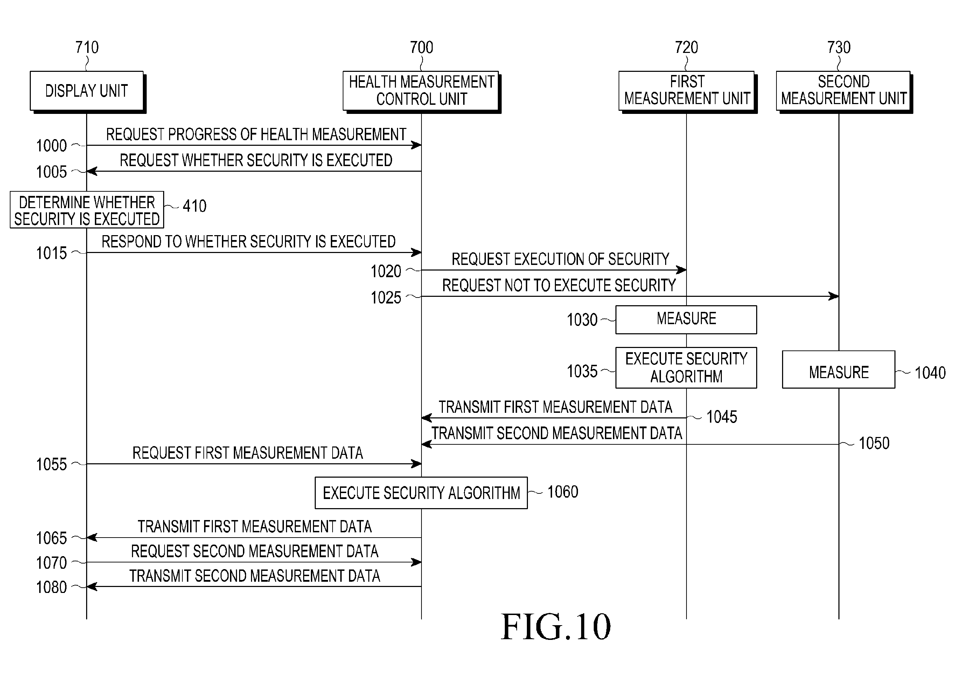

FIG. 10 is a flowchart for explaining an operation of a personal health record management system according to another embodiment of the present invention.

In the embodiment of FIG. 10, the display unit 710 may determine execution of a security on the measurement units. Referring to FIG. 10, if the user transmits a health measurement progress request related to the first and second measurement units 720 and 730 to the health measurement control unit 700 through the display unit 710 (1000), the health measurement control unit 700 requests information on execution of a security of the first and second measurement units 720 and 730 from the display unit 710 (1005). The display unit 710 determines such that a security is executed on the first measurement unit 720 and a security is not executed on the second measurement unit 730 (1010), and transmits the determined information to the health measurement unit 700 while a security execution response is carried in the determined information (1015). Then, the display unit 710 may transmit security related information on the first measurement unit 720 to the health measurement control unit 700, and the security related information may include information on the security algorithm executed by the first measurement unit 720. When requesting a health measurement progress from the health measurement unit 700, the display unit 710 may transmit information on execution of a security on the measurement units and security related information.

If receiving a security execution response, the health measurement control unit 700 requests the first measurement unit 720 to execute a security (1020) and the second measurement unit 730 not to execute a security (1025). If data on the health of the user is measured (1030), the first measurement unit 720 executes a security algorithm on the measurement data (1035), and transmits the first measurement data to the health measurement control unit 700 (1045). Because the second measurement unit 730 does not execute a security, it measures data on the health of the user (1040), and transmits the generated second measurement data to the health measurement control unit 700 without executing a security algorithm on the measured data (1050).

If the user requests first measurement data (1055), it is necessary to release a security to allow the user to read the first measurement data. When security related information is stored in the display unit 710, the health measurement control unit 700 may transmit the first measurement data to the display unit 710 without performing a separate security releasing step. The health measurement control unit 700 may execute a security algorithm on the first measurement data to release a security (1060), and transmit data whose security is released to the display unit 710 (1065). When the user requests the second measurement data (1070), the second measurement data is transmitted to the display unit 710 while a security algorithm is not executed because a security is not executed on the second measurement data.

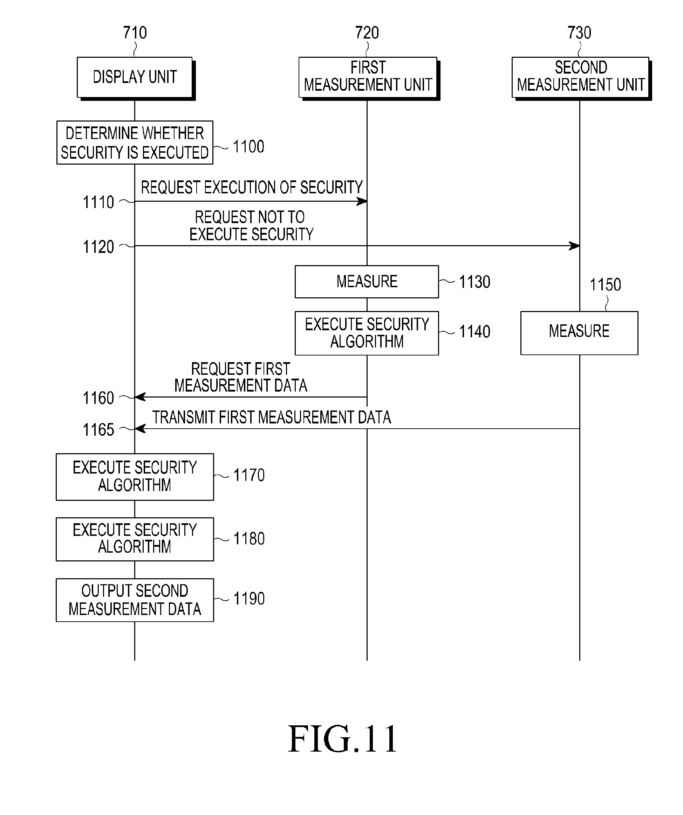

FIG. 11 is a flowchart for explaining an operation of a personal health record management system according to another embodiment of the present invention.

In the embodiment of FIG. 11, the display unit 710 may control execution of a security of the first and second measurement units 720 and 730. That is, if the user determines such that a security is executed on the first measurement unit 720 and a security is not executed on the second measurement unit 730 through the display unit (1100), the display unit 710 requests the first measurement unit 720 to execute a security (1110) and the second measurement unit 730 not to execute a security (1120). If data on the health of the user is measured (1130), the first measurement unit 720 executes a security algorithm on the measurement data (1140), and transmits the generated first measurement data to the display unit 710 (1160). Because the second measurement unit 730 does not execute a security, it measures data on the health of the user (1150), and transmits the generated second measurement data to the display unit 710 without executing a security algorithm on the measured data (1165).

When the user requests display of the measurement data, the display unit 710 releases the security by executing a security algorithm on the first measurement data because a security is executed on the first measurement data (1170) and outputs the measurement data whose security is released (1180), and the security algorithm is directly output while the security algorithm is not executed because a security is not executed on the second measurement data (1190).

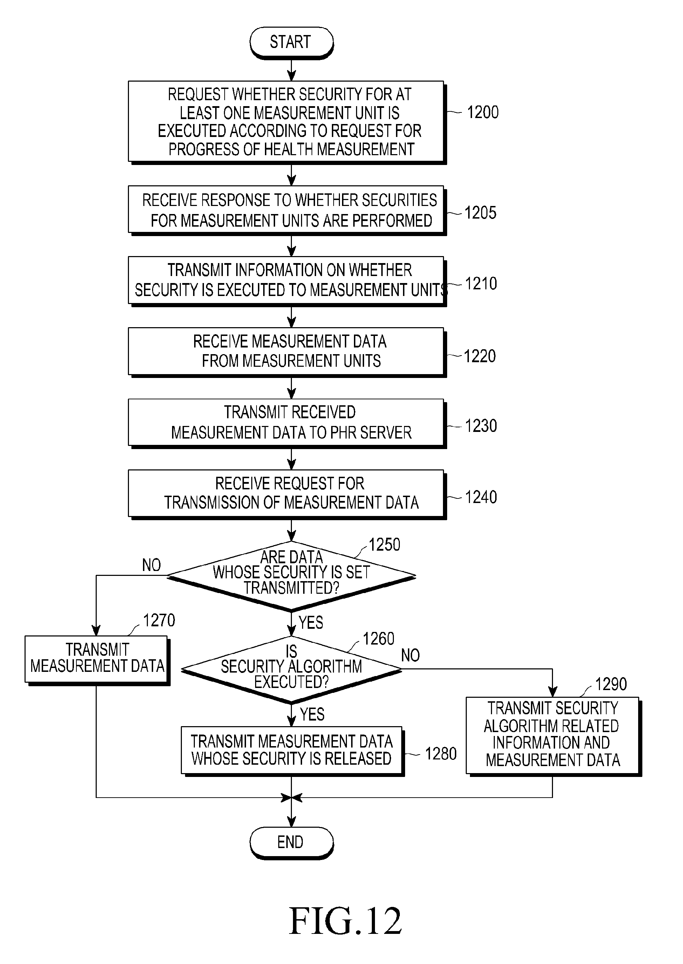

FIG. 12 is a flowchart showing an operation of a health measurement control apparatus of a personal health record management system according to an embodiment of the present invention.

Referring to FIG. 12, information on execution of a security on at least one measurement unit is requested from the user or the PHR server 750 according to a health measurement progress request of the user (1200), and a response on execution of a security of the measurement units is received (1205). Then, the measurement unit executing a security may receive security related information including information on the security algorithm executed by the measurement unit together.

If information on execution of a security is transmitted to the measurement units (1210) and data on the health of the user measured by the measurement units are received (1220), the received measurement data are transmitted to the PHR server 750 (1230). Meanwhile, when the user requests transmission of measurement data (1240) and the requested data are data whose security is set (Yes of 1250), if a security algorithm is executed (Yes of 1260), the measurement data whose security is released are transmitted to the user (1280), and if the security algorithm is not executed (No of 1260), measurement data and security algorithm related information are provided to the user (1290). When the requested data are not data whose security is not set (No of 1250), the measured data are transmitted without performing a security releasing step (1270).

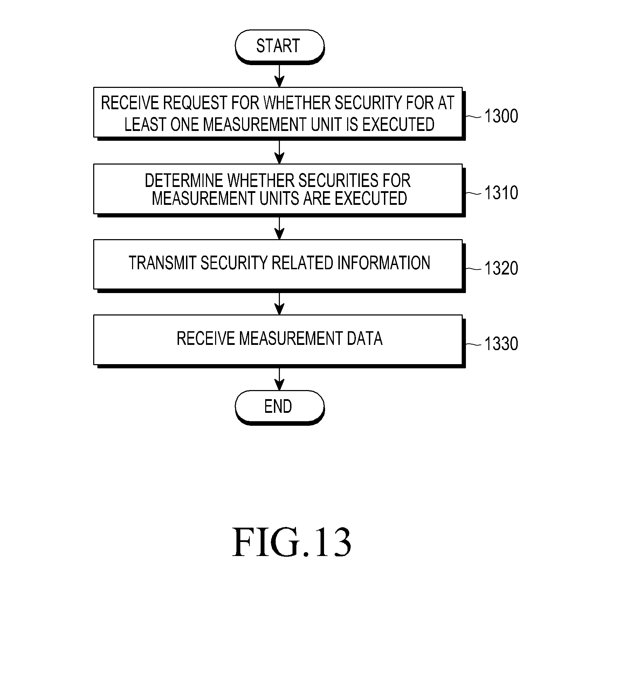

FIG. 13 is a flowchart showing an operation of a PHR server of a personal health record management system according to an embodiment of the present invention.

Referring to FIG. 13, a request for information on execution of a security on at least one measurement unit is received from the health measurement control unit 700 (1300), execution of a security on the measurement units is determined (1310) and security related information is transmitted to the health measurement control unit 700 (1320). Then, the measurement unit executing a security may transmit security related information including information on the security algorithm executed by the measurement unit together. Thereafter, health related data of the user measured by the health measurement control unit 700 are received (1330).

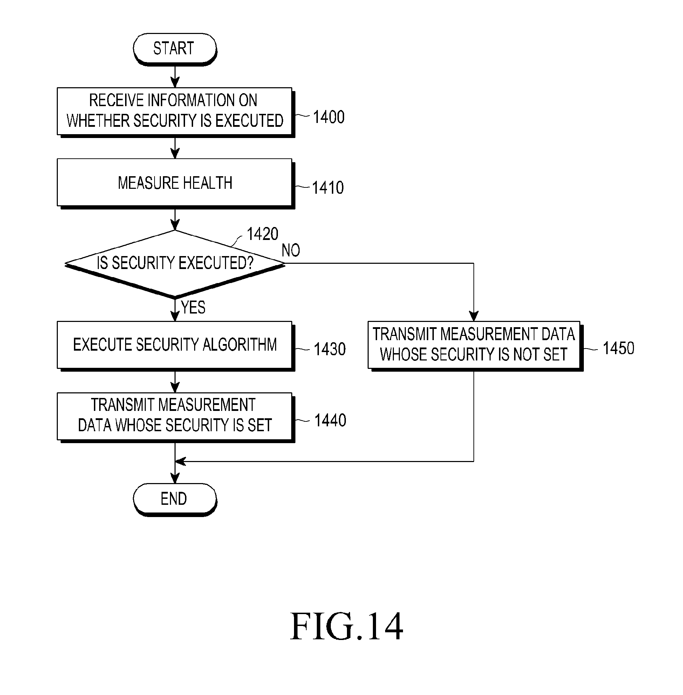

FIG. 14 is a flowchart showing an operation of a measurement apparatus of a personal health record management system according to an embodiment of the present invention. FIG. 14 exemplifies the first measurement unit 720, and may be applied to other measurement units including the second measurement unit 730 in the same way.

If information on execution of a security is received from the health measurement control unit 700 or the user, data on the health of the user are measured (1410) and a security of the measured data is set according to execution of a security. That is, when a security is executed (Yes of 1420), a security algorithm is executed (1430) and the measurement data whose security is set is transmitted to the health measurement control unit 700 or the user (1440), and if a security is not executed (No of 1420), measurement data whose security is not set are transmitted (1450).

According to the disclosed embodiment of the present invention, a requirement for a security of a health system can be satisfied by discriminatively providing a security algorithm of a higher level as compared with non-health devices using the same communication protocol to health devices dealing with secret information of the user. Further, a waste of power consumption in a gateway can be prevented by reducing power consumption due to driving of a security algorithm of a high level as much as possible.

In addition, according to the present invention, because the PHR server determines execution of a security of the measurement unit and manages security algorithms of the measurement units, the securities of the measurement units can be improved, and because execution of securities of the measurement units is determined according to situations, a resource can be efficiently used without performing a security whenever all the measurement units perform measurements.

Although the embodiment has been described in the detailed description of the present disclosure, the present disclosure may be modified in various forms without departing from the scope of the present disclosure. Therefore, the scope of the present disclosure is not limited to the embodiment described above, and should be defined by the accompanying claims and the equivalents of the claims.

## Claims

1. A method of providing a security by a gateway in a health system, the method comprising:
setting a communication link according to a first communication protocol with a communication device;
determining whether the communication device is a health device requiring a security of a high level;
if the communication device is a health device, performing a health security algorithm providing a security of a level higher than that of a security algorithm applied in the first communication protocol; and
performing a communication with the health device by using the health security algorithm.

2. The method of claim 1, wherein the determining comprises:
searching for identification information on the communication device from a data base (DB) in which information of health devices are registered in advance; and
if the identification information is present in the DB, determining that the communication device is the health device.

3. The method of claim 1, wherein the determining comprises:
extracting a profile on the communication device from a message received from the communication device; and
determining whether the communication device is the health device based on the profile information.

4. The method of claim 1, wherein the first communication protocol is at least one of WiFi, Bluetooth, and ZigBee.

5. A gateway apparatus for providing a security for a health system, the gateway apparatus comprising:
a transceiver for performing a communication with at least one communication device; and
a controller for, if a communication link according to a first communication protocol with the communication device is set, determining whether the communication device is a health device requiring a security of a high level, if the communication device is a health device, performing a health security algorithm providing a security of a level higher than that of a security algorithm applied in the first communication protocol, and performing a communication with the health device by using the health security algorithm.

6. The gateway apparatus of claim 5, wherein the controller searches for identification information on the communication device from a data base (DB) in which information of health devices are registered in advance, and if the identification information is present in the DB, determines that the communication device is the health device.

7. The gateway apparatus of claim 5, wherein the controller extracts a profile information on the communication device from a message received from the communication device, and determines whether the communication device is the health device based on the profile information.

8. The gateway apparatus of claim 5, wherein the first communication protocol is at least one of WiFi, Bluetooth, and ZigBee.

9. A method of managing a health record, the method comprising:
receiving first control information on whether security of at least one measurement apparatus is executed from a health record management server or a user;
transmitting second control information on whether security of the at least one measurement apparatus is executed to the at least one measurement apparatus based on the first control information; and
receiving data measured by the at least one measurement apparatus and generated based on the second control information and providing the data to the health record management server or the user.

10. The method of claim 9, wherein the first control information comprises information on a security algorithm executed by the at least one measurement apparatus.

11. The method of claim 9, wherein the providing of the data comprises, when a security is set in the data received from the measurement apparatus, releasing the security set in the received data and providing the data from which the security is released to the user.

12. The method of claim 9, wherein the providing of the data comprises, when a security is set in the data received from the measurement apparatus, providing information on a security algorithm executed by the at least one measurement apparatus to the user.

13. A method of managing a health record, the method comprising:
determining whether a security of at least one measurement apparatus is executed and generating control information on whether a security of the at least one measurement apparatus is executed;
transmitting the control information to the at least one measurement apparatus; and
receiving data measured by the at least one measurement apparatus and generated based on the control information.

14. The method of claim 13, wherein the control information comprises information on a security algorithm executed by the at least one measurement apparatus.

15. The method of claim 13, wherein the transmitting of the control information comprises transmitting the control information to a health measurement control apparatus for controlling the measurement apparatus.

16. The method of claim 13, further comprising, when the received data are data in which a security is set,
releasing the security of the received data; and
outputting data from which the security is released.

17. A method of managing a health record, the method comprising:
determining whether a security is executed on health data based on control information provided by a health record management server or a user;
measuring the health data;
executing a security algorithm to set a security in the measured health data according to the determination; and
transmitting the data in which the security is set to the health record management server or the user.

18. An apparatus for managing a health record, the apparatus comprising:
a transmitter that transmits a signal;
a receiver that receives a signal; and
a controller that transmits second control information on whether security of the at least one measurement apparatus is executed to the at least one measurement apparatus based on the first control information on whether security of at least one measurement apparatus is executed from a health record management server or a user, and receives data measured by the at least one measurement apparatus and generated based on the second control information and transmits the data to the health record management server or the user.

19. The method of claim 18, wherein the first control information comprises information on a security algorithm executed by the at least one measurement apparatus.

20. The method of claim 18, wherein, when a security is set in the data received from the measurement apparatus, the controller releases the security set in the received data and provides the data from which the security is released to the user.

21. The method of claim 18, wherein, when a security is set in the data received from the measurement apparatus, the controller provides information on a security algorithm executed by the at least one measurement apparatus to the user.

22. An apparatus for managing a health record, the apparatus comprising:
a transmitter for transmitting a signal;
a receiver that receives a signal;
a controller for determining whether a security is executed on at least one measurement apparatus and generating control information on whether a security is executed, and transmitting the control information to the at least one measurement apparatus.

23. The method of claim 22, wherein the control information comprises information on a security algorithm executed by the at least one measurement apparatus.

24. The method of claim 22, wherein the controller transmits the control information to a health measurement control apparatus for controlling the measurement apparatus.

25. The method of claim 22, wherein, when the received data are data in which a security is set, the controller releases the security of the received data and outputs the data from which the security is released.

26. An apparatus for managing a health record, the apparatus comprising:
a transmitter for transmitting a signal;
a receiver that receives a signal;
a controller for determining whether a security is executed on health data based on control information provided by a health record management server or a user, and executing a security algorithm to set a security in the measured health data according to whether a security is executed.
